# EUROPEAN PATENT APPLICATION

(11) **EP 3 851 075 A1**
(43) Date of publication of application: **21.07.2021**
(21) Application number: 19861118.8
(22) Date of filing: 09.09.2019
(51) Int. Cl.: A61F 2/16

(54) **INTRAOCULAR LENS INSERTION INSTRUMENT**

(30) Priority: 10.09.2018 JP 2018168903
(71) Applicant: KOWA COMPANY, LTD., Naka-ku Nagoya-shi Aichi 460-8625 (JP)
(72) Inventor: OBA, Norio, Hamamatsu-shi, Shizuoka 431-2103 (JP)
(74) Representative: Ribeiro, James Michael
(86) International application number: PCT/JP2019/035297
(87) International publication number: WO 2020/054641

(57) **Abstract**

An intraocular lens insertion apparatus which allows the intraocular lens to behave more stably is provided. The intraocular lens insertion apparatus includes a substantially tubular apparatus body which has, at a tip end thereof, an insertion tube part for inserting an intraocular lens in a folded state into an eye, the intraocular lens having a lens body and a support, a storage which stores the intraocular lens; and an extruding member which moves the intraocular lens in the insertion tube part by pushing the intraocular lens, the extruding member has a tip end part having a first protrusion protruding to one side in a left-right direction and a second protrusion protruding to the other side in the left-right direction, and during the movement of the intraocular lens in the insertion tube part by the extruding member, the first protrusion restricts a base end part of the support from moving to a front side of the intraocular lens in an optical-axis direction by abutting against the base end part from the front side in the optical-axis direction, and the second protrusion restricts a distal end part of the support from moving to the front side in the optical-axis direction by abutting against the distal end part from the front side in the optical-axis direction.

## Description

### [Field]

The present invention relates to an intraocular lens insertion apparatus.

### [Background]

In treatment of a cataract, an intraocular lens that is to be inserted as a substitute lens to replace a human opaque lens for refraction correction has become available. In an intraocular lens insertion surgery for cataract treatment, a few millimeter incision wound (an incision) is made, for example, at an edge of the cornea or sclera, and the eye lens is pulverized and removed from the incision by phacoemulsification, and then the intraocular lens is inserted and fixed.

In recent years, when an intraocular lens is inserted into an eye through an incision, an insertion apparatus having a cartridge, which is preloaded with an intraocular lens, may be used. An operator inserts the tip opening of the insertion tube part provided at the tip end of the insertion apparatus body into the eye through the incision. The operator inserts the intraocular lens into the eye by pushing the intraocular lens out from the tip opening of the insertion tube part with a rod-like plunger provided in the insertion apparatus.

In general, an intraocular lens has a lens body and supports that are connected to the lens body. When the intraocular lens is inserted into an eye using the above insertion apparatus, the intraocular lens is in a state of being folded in accordance with the inner wall shape of the insertion tube part during movement of the intraocular lens by the plunger. At that time, merely pushing out the intraocular lens with the plunger the intraocular lens may not stably fix the orientation of the intraocular lens within the insertion tube part, and the behavior of the intraocular lens may not be stable. Therefore, there has been proposed a technology of providing to the tip end of the plunger a configuration for stabilizing the behavior of the intraocular lens in the insertion tube part (see PTL 1 to PTL 3).

### [Citation List]

### [Patent Literature]

[PTL 1] Japanese Laid-open Patent Publication No.2012-50713
[PTL 2] Japanese Laid-open Patent Publication No.H05-103803
[PTL 3] Japanese Patent No. 5570254

### [Summary]

### [Technical Problem]

However, in the conventional intraocular lens insertion apparatuses, the positions of the supports for the intraocular lens may change when the intraocular lens is moved by the plunger in the insertion tube part. In such a case, the intraocular lens may exhibit abnormal behavior within the insertion tube part even using the intraocular lens insertion apparatus above.

With the foregoing in view, it is an object of the present disclosure to provide an intraocular lens insertion apparatus which allows the intraocular lens to behave more stably.

### [Solution to Problem]

An intraocular lens insertion apparatus according to the disclosure includes a substantially tubular apparatus body which has, at a tip end thereof, an insertion tube part for inserting an intraocular lens in a folded state into an eye, the intraocular lens having a lens body and a long support which is connected at one end to the lens body, a storage which stores the intraocular lens, and an extruding member which slides in the apparatus body and moves the intraocular lens in the insertion tube part by pushing the intraocular lens, wherein the extruding member has a tip end part having a first protrusion protruding to one side in a left-right direction and a second protrusion protruding to the other side in the left-right direction in a front view of the extruding member in a direction in which the intraocular lens is extruded, during the movement of the intraocular lens in the insertion tube part by the extruding member, the first protrusion restricts a base end part of the support from moving to a front side of the intraocular lens in an optical-axis direction by abutting against the base end part from the front side in the optical-axis direction, and the second protrusion restricts a distal end part of the support nearer to a distal end thereof than the base end part from moving to the front side in the optical-axis direction by abutting against the distal end part from the front side in the optical-axis direction. In this way, when the intraocular lens is released into the eye from the insertion apparatus, the movement of the base end part and the distal end part of the support is restricted, and the intraocular lens can behave more stably.

In the intraocular lens insertion apparatus, the extruding member has a notch positioned at a front side of the optical-axis direction than the intraocular lens stored in the storage, and the notch may support an intermediate part of the support between the distal end part and the base end part from a rear side in the optical-axis direction in a state where the intraocular lens is stored in the storage. In the intraocular lens insertion apparatus, in a state where the intraocular lens is stored in the storage, the first protrusion may be formed so that the base end part of the support can move to the front side in the optical-axis direction between the first protrusion and the apparatus body, and the second protrusion may be formed so that the distal end part of the support can move to the front side in the optical-axis direction between the second protrusion and the apparatus body.

In the intraocular lens insertion apparatus, in a state where the tip end part of the extruding member is at the insertion tube part of the apparatus body, a first gap through which the base end part of the support can be inserted may be formed between the first protrusion and the insertion tube part, and a second gap through which the distal end part of the support can be inserted may be formed between the second protrusion and the insertion tube part, and when the intraocular lens is moved into the insertion tube part from the storage by the extruding member, the base end part of the support may enter the first gap and the distal end part of the support may enter the second gap.

In the intraocular lens insertion apparatus, a width of a part where the first protrusion and the second protrusion are provided in the left-right direction may be substantially equal to a width of at least a part of the insertion tube part in the left-right direction. In the intraocular lens insertion apparatus, the notch of the extruding member may be provided nearer to a base end of the apparatus body than the first protrusion and the second protrusion.

### [Advantageous Effects of Invention]

According to the present disclosure, an intraocular lens insertion apparatus which allows the intraocular lens to behave more stably can be provided.

### [Brief Description of Drawings]

[Fig. 1] Figs. 1(a) and 1(b) are diagrams illustrating the general structure of an intraocular lens according to an embodiment of the invention.
[Fig. 2] Figs. 2(a) and 2(b) are diagrams illustrating the general structure of the intraocular lens insertion apparatus according to the embodiment.
[Fig. 3] Fig. 3 is a diagram illustrating the general structure of a nozzle body according to the embodiment.
[Fig. 4] Figs. 4(a) and 4(b) are diagrams illustrating the general structure of a positioning member according to the embodiment.
[Fig. 5] Figs. 5(a) and 5(b) are diagrams illustrating the general structure of a plunger according to the embodiment.
[Fig. 6] Figs. 6(a) to 6(f) are enlarged diagrams illustrating the working part of the plunger according to the embodiment.
[Fig. 7] Fig. 7 is a diagram illustrating a state of the working part of the plunger and an intraocular lens according to the embodiment.
[Fig. 8] Fig. 8 is a diagram illustrating a state of the working part of the plunger and the intraocular lens according to the embodiment
[Fig. 9] Fig. 9 is a sectional diagram illustrating the positional relation among the tip end of a nozzle body, the plunger, and the support for the intraocular lens.

### [Description of Embodiments]

Hereinafter, an embodiment of the present invention is described with reference to drawings.

First, an intraocular lens 2 to be inserted into the patient's eye using the intraocular lens insertion apparatus 1 according to the embodiment is described before describing the intraocular lens insertion apparatus 1.

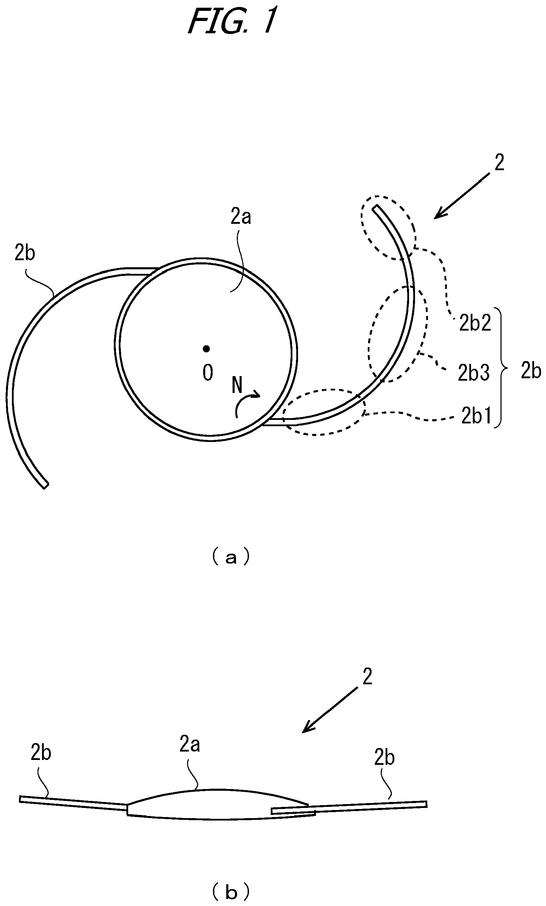

Fig. 1 illustrates the general structure of the intraocular lens 2 according to the embodiment. Fig. 1(a) is a plan view, and Fig. 1(b) is a side view.

The intraocular lens 2 includes a lens body 2a having predetermined refraction as illustrated in Fig. 1, and two long supports 2b connected to the lens body 2a to hold the lens body 2a in the eye. As illustrated in Fig. 1, the intraocular lens 2 is a so-called three-piece type, and the lens body 2a and the supports 2b are formed discretely, both of which are made of a flexible resin material.

In the following description, among the both sides in the optical-axis direction of the intraocular lens 2, the side facing the cornea after insertion of the lens into the eye is defined as the "front side in the optical-axis direction," and the opposite side is defined as the "rear side in the optical-axis direction". A part of the support 2b is defined as a "base end part 2b1," a part closer to the distal end than the base end part 2b1 is defined as a "distal end part 2b2," and the part between the base end part 2b1 and the distal end part 2b2 is defined as an "intermediate part 2b3". In other words, the base end part 2b1 is located nearer to the lens body 2a than the distal end part 2b2. Note that the base end part 2b1 does not need to include the connection part of the support 2b with the lens body 2a. Similarly, it should be noted that the distal end part 2b2 does not need to include the distal end of the support 2b.

Next, the general structure of the intraocular lens insertion apparatus 1 is described.

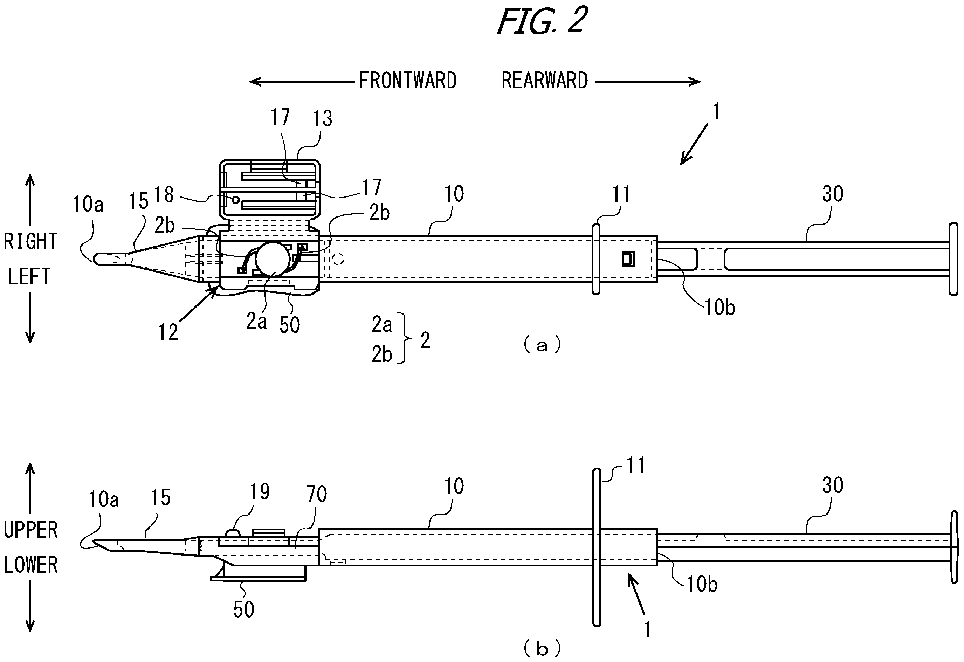

Fig. 2 illustrates the general structure of the intraocular lens insertion apparatus 1 according to the embodiment. Fig. 2(a) is a plan view in a state where a stage lid part 13 is opened, which will be described, and Fig. 2(b) is a side view in a state where the stage lid part 13 is closed.

The intraocular lens insertion apparatus 1 includes a substantially tubular nozzle body 10, a storage 70 for storing the intraocular lens 2, and a plunger 30 configured to be slidable within the nozzle body 10 to push the intraocular lens 2 out of the nozzle body 10. Note that the nozzle body 10 is an example of the apparatus body, and the plunger 30 is an example of the extruding member.

The nozzle body 10 of the intraocular lens insertion apparatus 1 is a tubular member having a substantially rectangular cross-section as a whole and has a tip end part 10a provided at the tip end side, a rear end part 10b provided at the rear end side, and a nozzle part 15 provided between the tip end part 10a and the rear end part 10b. The tip end part 10a is an example of an insertion tube part. As illustrated in Fig. 2(b), the tip end part 10a is in a substantially cylindrical shape having such a small diameter that the tip end can be inserted into a small incision in the eye, and its end part opens obliquely with respect to the extending direction of the nozzle body 10. The rear end part 10b is provided with a large opening through which the plunger 30 can be inserted. The nozzle part 15 is in contact with the tip end part 10a and formed to have a shape gradually tapered from the rear end part 10b to the tip end part 10a.

The storage 70 is provided between the nozzle part 15 of the nozzle body 10 and the rear end part 10b and is integrally formed with the nozzle body 10. The storage 70 includes a stage part 12 for setting the intraocular lens 2 thereon and a stage lid part 13 for covering the stage part 12. In cooperation with a positioning member 50 which will be described, the storage 70 allows the intraocular lens 2 to be held stably in the storage 70.

The plunger 30 includes a working part 31 in abutment against the intraocular lens 2 provided on the tip end side and an insertion part 32 provided on the rear end side and operated by the operator. The plunger 30 is configured to reciprocating motion within the nozzle body 10 and is used to push the intraocular lens 2 out of the tip end part 10a into the eye.

Note that the nozzle body 10, the storage 70, the plunger 30, and the positioning member 50 of the intraocular lens insertion apparatus 1 are made of a resin material such as polypropylene. Polypropylene has been proven useful in the field of medical equipment and is a highly reliable material for example in terms of chemical resistance.

In the following description, the direction (side) from the rear end part 10b to the tip end part 10a of the nozzle body 10 is defined as the "frontward (side)," the opposite direction (side) thereto is defined as the "rearward (side)," the direction (side) in front of the paper sheet surface in Fig. 1(a) is defined as the "upward (side)," the opposite direction (side) thereto is defined as the "downward (side)," the direction (side) in front of the paper sheet surface in Fig. 1(b) is defined as the "leftward (side)," and the opposite direction (side) thereto is defined as the "rightward (side)". In this case, the intraocular lens 2 is set and stored in the storage 70 such that the front side in the optical-axis direction faces upward and the rear side in the optical-axis direction faces downward with respect to the stage part 12 as will be described. In this sense, the "front side in the optical-axis direction" and the simple "front side" are different and the "rear side in the optical-axis direction" and the simple "rear side" are different. The direction in which the plunger 30 reciprocates is defined as the "extruding direction." In this case, the plunger 30 reciprocates in the front-rear direction in the nozzle body 10.

Next, the structure of the nozzle body 10 and the storage 70 is described.

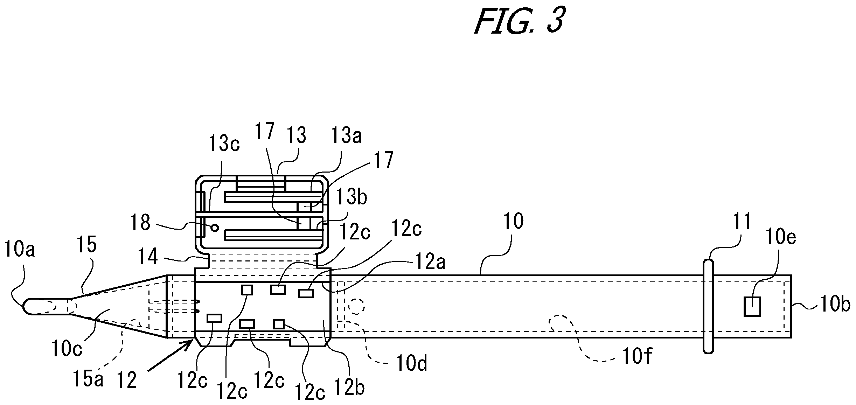

Fig. 3 is a view of the general structure of the nozzle body 10 according to the embodiment.

A through hole 10c on the tip end side and a through hole 10f on the rear end side are formed inside the nozzle body 10, and these holes have cross-sectional shapes changed according to change in the outer shape of the nozzle body 10. The through hole 10c is a hole which forms a part of the travel path in which the intraocular lens 2 is moved under pressure, and the through hole 10f is a hole through which the plunger 30 is inserted. The intraocular lens 2 is deformed and folded into a shape which allows the lens to enter easily into the incision wound of the eye according to change in the cross-sectional shape of the through hole 10c and is released into the eye.

The tip end part 10a of the nozzle body 10 has a shape which is slanted so that the upper front end of the nozzle body 10 is ahead of the lower front end. Note that the tip end part 10a may be linearly obliquely cut when viewed from the left-right direction or may be cut slantingly so that the tip end part 10a is raised outward or curved. The oblique cutting of the tip end part 10a makes it easier for the operator to insert the tip end part 10a into the incision wound of the eye compared with otherwise. The nozzle body 10 also has a plate-shaped projecting hold part 11 near the rear end part 10b. The operator places the finger on the hold part 11 in pushing the plunger 30 toward the tip end of the nozzle body 10.

Meanwhile, the storage 70 stores the intraocular lens 2 as described above and is integrally provided with the nozzle body 10 on the rear side of the nozzle part 15 of the nozzle body 10. As described above, the storage 70 includes a stage part 12 for setting the intraocular lens 2 thereon and the stage lid part 13 for covering the stage part 12. After the intraocular lens 2 is set at the stage part 12, as the stage lid part 13 is closed, the intraocular lens 2 is stored in the storage 70.

A stage groove 12a having a width slightly larger than the diameter of the lens body 2a of the intraocular lens 2 is formed at the stage part 12 of the storage 70. The size of the stage groove 12a in the front-rear direction is set to be larger than the maximum width of the intraocular lens 2 including the supports 2b and 2b which extend on the both sides of the intraocular lens 2. A set surface 12b is formed by the bottom surface of the stage groove 12a. The position of the set surface 12b in the up-down direction is set above the height position of the bottom surface of the through hole 10f of the nozzle body 10, and the set surface 12b and the bottom surface of the through hole 10f are connected to each other by a bottom slope 10d. The set surface 12b of the stage part 12 is processed so that the positioning member 50 can be removed from the lower side thereof.

Here, the positioning member 50 is described in detail.

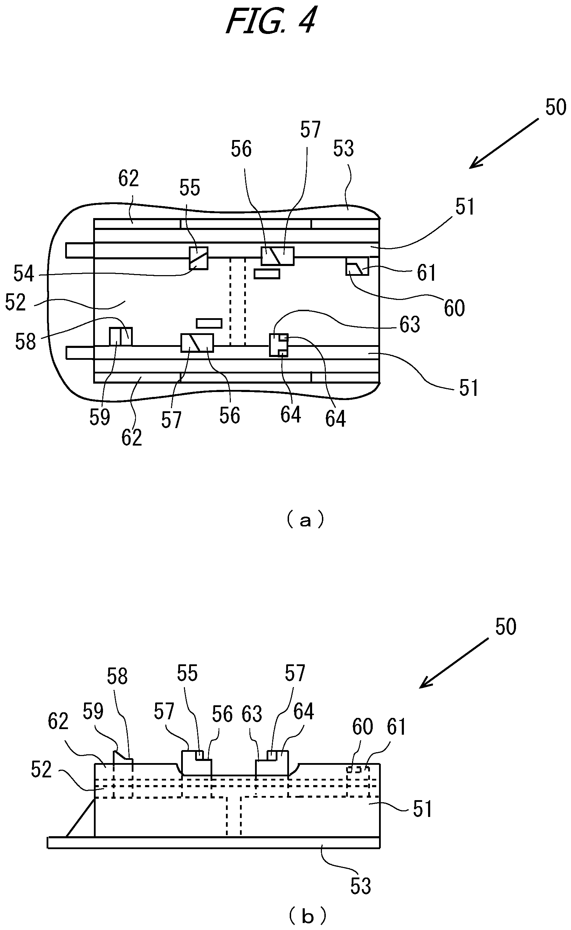

Fig. 4 illustrates the general structure of the positioning member 50 according to the embodiment. Fig. 4(a) is a plan view, and Fig. 4(b) is a left side view. It is assumed that the intraocular lens 2 in the following description is stored in the storage 70. In the following description, among the two supports 2b and 2b for the intraocular lens 2 stored in the storage 70, the support 2b placed on the front side of the lens body 2a may be referred to as the "front support" and the support placed on the rear side of the lens body 2a may be referred to as the "rear support."

The positioning member 50 is formed discretely from the nozzle body 10 and has a pairs of side walls 51 connected by a connecting part 52. A holding part 53 which expands outwardly is formed at the lower ends of the side walls 51.

First mounting part 54 and second mounting part 63 which protrude upwardly are formed inside the side walls 51 and 51. A first positioning part 55 is formed to protrude on the outer periphery side of the upper end surface of the first mounting part 54. A pair of second positioning parts 64 and 64 are formed to protrude at the upper end surface of the second mounting part 63 to position the lens body 2a and the support 2b of the intraocular lens 2. The isolation distance between the first positioning part 55 and the second positioning parts 64 and 64 is set slightly larger than the diameter of the lens body 2a of the intraocular lens 2.

A pair of third mounting parts 56 and 56 which protrude upwardly are formed inside the side walls 51 and 51. The upper surfaces of the first mounting part 54, the second mounting part 63, and the third mounting parts 56 and 56 are at the same height. Third positioning parts 57 and 57 which protrude further upwardly are formed at the outer parts of the upper surfaces of the third mounting parts 56 and 56 entirely in the left-right direction of the third mounting parts 56 and 6. The isolation distance between the third positioning parts 57 and 57 are set to be slightly larger than the diameter measurement of the lens body 2a of the intraocular lens 2.

A fourth mounting part 58 on which a part of one of the supports 2b of the intraocular lenses 2 is mounted is formed inside the side walls 51 and 51. A fourth positioning part 59 is formed to further protrude upwardly from the fourth mounting part 58. A part of the front support 2b abuts against the fourth positioning part 59. A fifth mounting part 60 on which a part of the rear support 2b of the intraocular lens 2 is mounted is formed inside the side walls 51 and 51. A fifth positioning part 61 is formed to further protrude upwardly from the fifth mounting part 60. A part of the rear support 2b abuts against the fifth positioning part 61.

As illustrated in Fig. 4(b), the heights of the upper surfaces of the fifth mounting part 60 and the fifth positioning part 61 are lower than the heights of the upper surfaces of the first to fourth mounting parts 54, 63, 56, and 58 and the first to fourth positioning parts 55, 64, 57, and 59. Rotation preventing walls 62 are provided on the outside of the side walls 51 and 51 to prevent unwanted rotation when the positioning member 50 is removed.

Meanwhile, set surface through holes 12c are formed through the set surface 12b in a thickness-wise direction at the set surface 12b of the nozzle body 10. When viewed from above, the outer shapes of the set surface through holes 12c are similar to and slightly larger than the shapes of the first to fifth mounting parts 54, 63, 56, 58, and 60 and the first to fifth positioning parts 55, 64, 57, 59, and 61 of the positioning member 50.

The positioning member 50 configured in this manner can be attached to the nozzle body 10 as the positioning member 50 is inserted from the lower side of the set surface 12b to the set surface through holes 12c until the first to fifth mounting parts 54, 63, 56, 58, and 60 and the first to fifth positioning parts 55, 64, 57, 59, and 61 project above the set surface 12b.

The bottom surface of the outer periphery of the lens body 2a of the intraocular lens 2 is mounted on the upper surfaces of the first mounting part 54, the second mounting part 63, and the third mounting parts 56 and 56, and the two supports 2b are mounted on the upper surfaces of the fourth mounting part 58 and the fifth mounting part 60. In this way, the lens body 2a has its position restricted in the horizontal direction (a direction horizontal to the set surface 12b) by the first positioning part 55, the second positioning parts 64 and 64, and the third positioning parts 57 and 57, and the two supports 2b have their positions restricted in the horizontal direction by the fourth positioning part 59 and the fifth positioning part 61. In this way, the storage 70 can stably hold the intraocular lens 2 in cooperation with the positioning member 50.

The foregoing is a detailed description of the positioning member 50.

Meanwhile, the stage lid part 13 has a checking window part 17 as a thin wall part thereof. Here, the degree of how thin the checking window part 17 should be may be determined as appropriate on the basis of the material used for forming the stage lid part 13 and the visibility of the intraocular lens 2 from the checking window part 17. The presence of the checking window part 17 may also reduce contraction when the stage lid part 13 is formed. The stage lid part 13 is provided with a lubricant supply hole 18 for injecting a viscoelastic material as a lubricant into the stage part 12 before the operation of inserting the intraocular lens 2 into the eye (hereinafter simply referred to as "pre-surgery") and a guide wall 19 for guiding an injecting member such as a needle for injecting the viscoelastic material into the lubricant supply hole 18. The lubricant supply hole 18 is a hole which communicates between the inside and outside of the storage 70 when the stage lid part 13 is closed. The guide wall 19 is provided to surround at least a part of the periphery of the lubricant supply hole 18. The operator moves the tip of the injecting member for injecting the viscoelastic material to the lubricant supply hole 18 while keeping the tip end of the injecting member in abutment against the guide wall 19. In this way, the guide wall 19 facilitates introduction of the injecting member into the lubricant supply hole 18.

The stage lid part 13 is integrally formed with the stage part 12. The stage lid part 13 has a size equivalent to that of the stage part 12 in the front-rear direction. The stage lid part 13 is connected by a thin plate-like connection part 14 formed by a side surface of the stage part 12 extending toward the stage lid part 13. The connection part 14 is flexibly formed at the center part, and the stage lid part 13 can overlap and close the stage part 12 from above by bending the connection part 14. The stage lid part 13 is provided with ribs 13a and 13b on the surface opposed to the set surface 12b when the lid part is closed, for reinforcing the stage lid part 13 and stabilizing the position of the intraocular lens 2. A guide projection 13c is also provided as an upper side guide for the plunger 30.

Next, the structure of the plunger 30 is described.

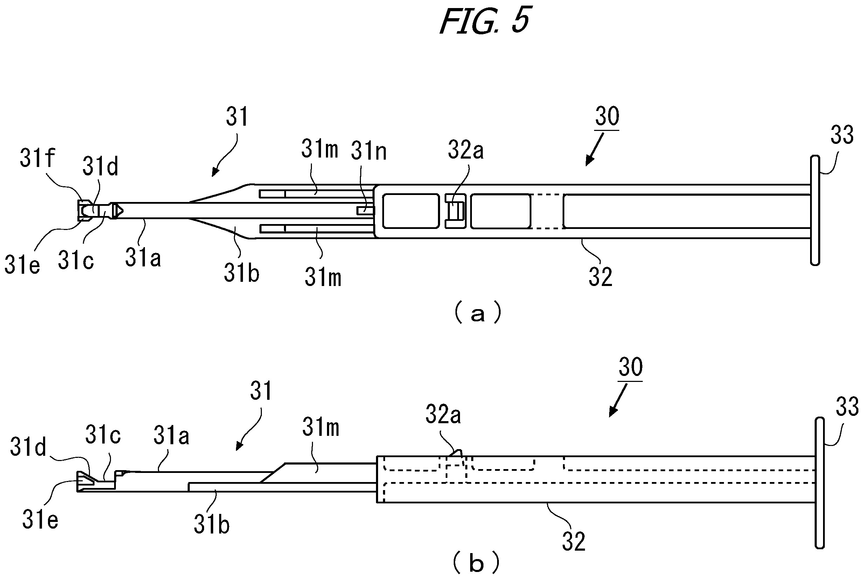

Fig. 5 illustrates the general structure of the plunger 30 according to the embodiment. Fig. 5(a) is a plan view, and Fig. 5(b) is a side view.

The plunger 30 has a slightly greater length than the nozzle body 10 in the front-rear direction. The plunger 30 is formed by a basically cylindrical working part 31 and a basically rectangular rod-shaped insertion part 32 on the rear end side. Note that the working part 31 is an example of the tip end of the extrusion member.

The working part 31 includes a cylindrical part 31a and a thin plate-like flat part 31b expanding from the cylindrical part 31a in the left-right direction. The flat part 31b is provided with a pair of sliding parts 31m capable of sliding on the inner wall surface of the nozzle body 10 as the plunger 30 moves in the nozzle body 10. As the sliding parts 31m slide on the inner wall surface of the nozzle body 10, frictional force acting as resistance in operating the plunger 30 is generated. This allows the operator to perform stable extruding operation to the plunger 30. The flat part 31b is also provided with a protrusion 31n which can come into abutment against the inner wall surface of the nozzle body 10. The protrusion 31n abuts against the inner wall surface of the nozzle body 10 to restrain the frontward movement of the plunger 30 within the nozzle body 10. In this way, the operator can be prevented from pushing the plunger 30 too frontward in the nozzle body 10.

Meanwhile, the insertion part 32 has a generally H-shaped cross section as a whole and has its sizes in the left-right and up-down directions set to be slightly smaller than that of the through hole 10f of the nozzle body 10. A disk-like pressure plate part 33 which extends in the up-down and left-right directions is formed at the rear end of the insertion part 32. A claw part 32a is provided at the part of the insertion part 32 ahead of the midpoint in the front-rear direction to project upward from the insertion part 32, and the claw part 32a can move vertically by the elasticity of the material of the plunger 30. When the plunger 30 is inserted into the nozzle body 10, an engaging hole 10e illustrated in Fig. 3 provided in the thickness-wise direction on the upper surface of the nozzle body 10 and the claw part 32a are engaged, so that the relative position of the nozzle body 10 and the plunger 30 in the initial state is determined. The claw part 32a and the engaging hole 10e are formed in such positions that in the engaged state, the tip end of the working part 31 is positioned at the rear side of the lens body 2a of the intraocular lens 2 set at the stage part 12, and a notch 31c is positioned so that the rear support 2b of the intraocular lens 2 can be supported from the rear side of the intraocular lens 2 in the optical-axis direction.

Next, the structure of the working part 31 of the plunger 30 is described in more detail.

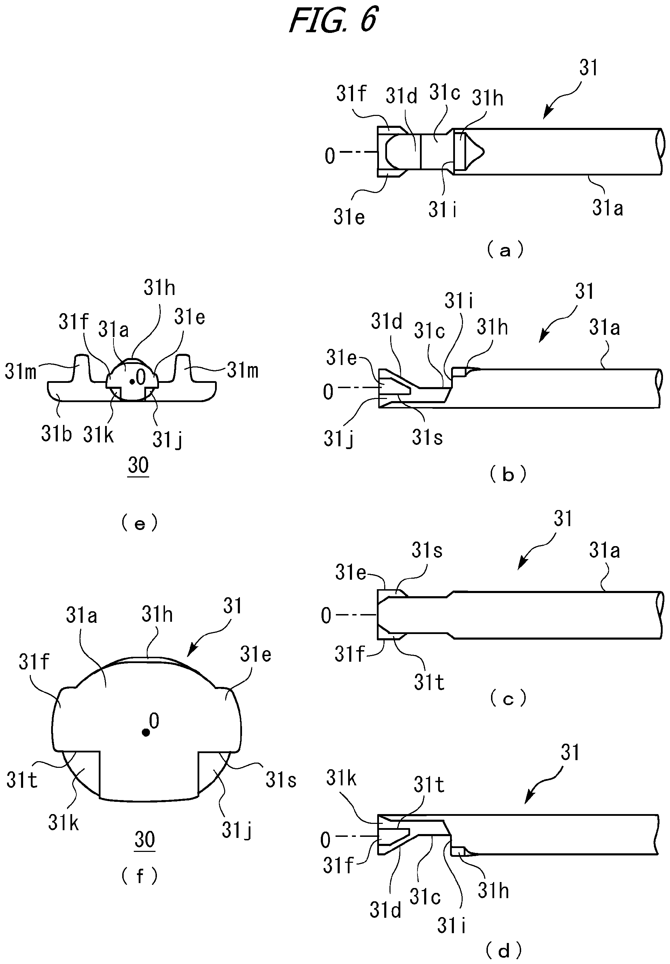

Fig. 6 illustrates in enlarged views, the working part 31 of the plunger 30 according to the embodiment. Fig. 6(a) is a top view, Fig. 6(b) is a left side view, Fig. 6(c) is a bottom view, Fig. 6(d) is a right side view, Fig. 6(e) is a front view, and Fig. 6(f) is a front view of a further enlargement of Fig. 6(e).

As illustrated in Figs. 6(a) and 6(b), the cylindrical part 31a of the working part 31 has the groove-like notch 31c which opens to the upper side of the working part 31 and passes in the left-right direction. The notch 31c is positioned more on the front side in the optical-axis direction than the intraocular lens 2 set in the stage part 12. As illustrated in Fig. 6(b), the groove wall 31d on the tip end side of the notch 31c is formed as an upwardly inclined surface toward the tip end side of the working part 31.

As illustrated in Fig. 6(f), protrusions 31e and 31f is provided in the working part 31. The protrusions 31e and 31f project from the cylindrical part 31a in the left-right direction in the front view of the plunger 30 in the extrusion direction (the extended direction of the central axis O) of the plunger 30. The protrusions 31e and 31f are locally raised parts in a direction away from the central axis O from the outer peripheral surface of the cylindrical part 31a. Note that the protrusion 31e is an example of the first protrusion, and the protrusion 31f is an example of the second protrusion. The presence of the protrusions 31e and 31f allows gaps 31j and 31k to be formed under the protrusions 31e and 31f and between the protrusions 31e and 31f and the nozzle body 10. The gaps 31j and 31k extend from the tip end surface of the working part 31 to the notch 31c along the sides of the working part 31 of the plunger 30 as illustrated in Fig. 6(b) and Fig. 6(d).

The lower side surfaces of the protrusions 31e and 31f form abutment surfaces 31s and 31t in abutment against the supports 2b in the tip end part 10a of the nozzle body 10. As illustrated in Fig. 6(f), the shapes of the protrusions 31e and 31f in the front view of the working part 31 may be substantially symmetrical. The gap 31j is an example of the first gap, and the gap 31k is an example of the second gap.

Here, according to the embodiment, the width of the part of the working part 31 in the left-right direction where the protrusions 31e and 31f are provided should be set with the following points in mind.

More specifically, when the protrusions 31e and 31f are positioned at the storage 70, an empty space is provided between the protrusion 31e and the stage groove 12a so that the base end part 2b1 of the rear support 2b can be moved frontward in the optical-axis direction in the space. Similarly, an empty space is provided between the protrusion 31f and the stage groove 12a so that the distal end part 2b2 of the rear support 2b can be moved frontward in the optical-axis direction in the space.

When the protrusions 31e and 31f are positioned at the tip end part 10a, the base end part 2b1 of the rear support 2b is prevented from moving frontward in the optical-axis direction between the protrusion 31e and the inner wall surface of the tip end part 10a. Similarly, the distal end part 2b2 of the rear support 2b is prevented from moving frontward in the optical-axis direction between the protrusion 31f and the inner wall surface of the tip end part 10a. For this reason, the protrusions 31e and 31f are formed such that the width of the part in the left-right direction where the protrusions 31e and 31f are provided has a width substantially the same as the width of at least a part of the tip end part 10a in the left-right direction.

Next, the behavior of the intraocular lens 2 when the intraocular lens insertion apparatus 1 is used is described.

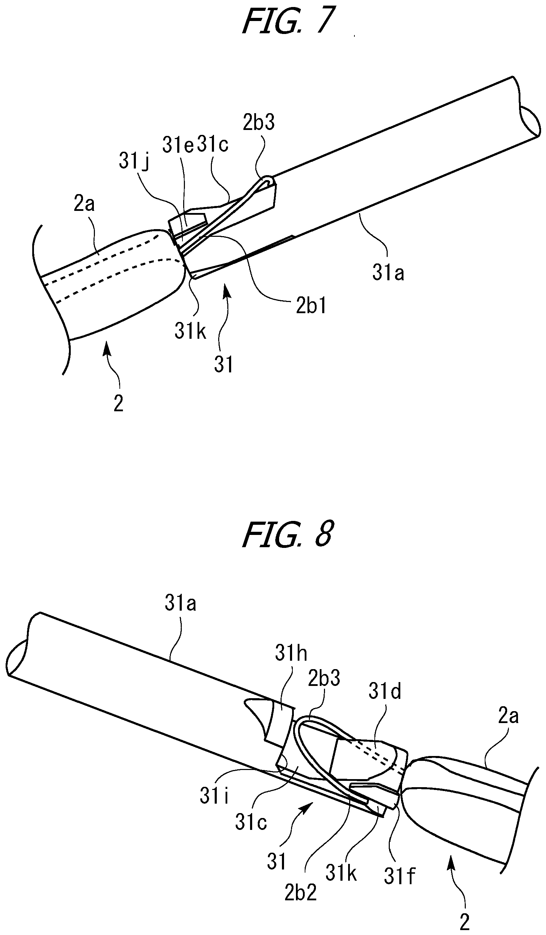

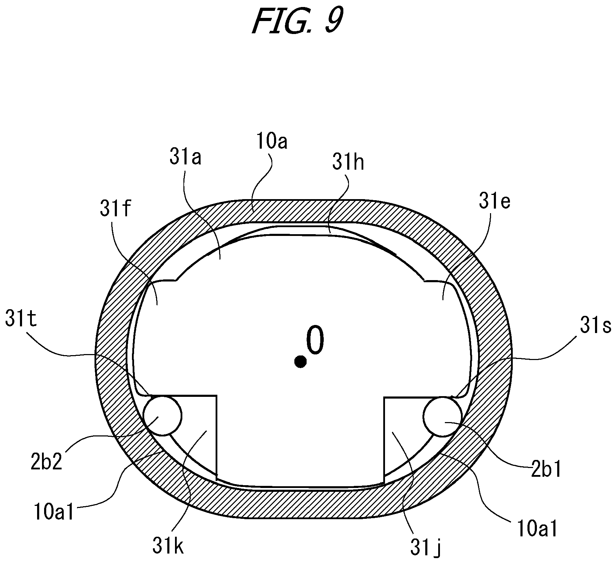

Figs. 7 and 8 are views of the working part 31 of the plunger 30 and the intraocular lens 2 according to the embodiment. Fig. 9 is a cross-sectional view illustrating the positional relation between the tip end part 10a of the nozzle body 10, the plunger 30, and the supports 2b for the intraocular lens 2 according to the embodiment. These views illustrate how the intraocular lens 2 moves in the tip end part 10a, and Fig. 7 and Fig. 8 does not illustrate the nozzle body 10 for ease of understanding.

The intraocular lens insertion apparatus 1 according to the embodiment is a so-called preset type apparatus.

More specifically, during the manufacture of the intraocular lens insertion apparatus 1, while the stage lid part 13 is opened and the positioning member 50 is mounted to the stage part 12, the intraocular lens 2 is set so that the front side in the optical-axis direction is on the upper side with respect to the stage part 12. Then, after the stage lid part 13 is closed, the intraocular lens insertion apparatus 1 is shipped and sold.

While the intraocular lens 2 is stored in the storage 70, the intermediate part 2b3 of the rear support 2b is supported by the notch 31c. At the time, the base end part 2b1 and the distal end part 2b2 of the rear support 2b have their positions unrestricted by the protrusions 31e and 31f, and are allowed to move frontward in the optical-axis direction.

Subsequently, the operator using the intraocular lens insertion apparatus 1 wets the intraocular lens 2 with a viscoelastic material or perfusion solution while the stage lid part 13 is kept closed and then removes the positioning member 50. In this way, this removes the positional restrictions on the intraocular lens 2 in the storage 70 in the horizontal direction.

Subsequently, the operator pushes the plunger 30 toward the tip end of the nozzle body 10. In this way, the tip end surface of the working part 31 of the plunger 30 comes into abutment against the rear side of the outer periphery of the intraocular lens 2, and the intraocular lens 2 moves from the storage 70 to the nozzle part 15. The intraocular lens 2 moves through the nozzle part 15, and during the movement, the lens body 2a is gradually folded into a valley shape for example according to the shape of the inner walls of the nozzle part 15. At the time, the part of the rear support 2b for the intraocular lens 2 which is closer to the lens body 2a moves to the vicinity of the lower end of the tip end surface of the working part 31. Here, the lower end of the tip end surface of the working part 31 is the end of the tip end surface positioned behind the intraocular lens 2 stored in the storage 70 in the optical-axis direction.

Subsequently, when the operator further pushes the plunger 30, the base end part 2b1 of the rear support 2b of the intraocular lens 2 enters the gap 31j as illustrated in Fig. 7. Then, when the base end part 2b1 enters the gap 31j, the part of the rear support 2b nearer to the distal end than the part held in the gap 31j extends to the notch 31c. The rear support 2b extending to the notch 31c is curved by a wall part 31i and a convex part 31h as illustrated in Fig. 8, so that the rear support 2b can be prevented from advancing rearward of the cylindrical part 31a. When the rear support 2b is curved by the wall part 31i and the convex part 31h, the tip end of the rear support 2b is directed toward the gap 31k. In this state, the rear support 2b extends further to the notch 31c, and the tip end part 2b2 of the rear support 2b, i.e., the part of the rear support 2b positioned further from the lens body 2a enters the gap 31k from the notch 31c.

In the process in which the plunger 30 is pushed in, the gap between the protrusions 31e and 31f and the inner wall surface of the nozzle body 10 becomes gradually narrower in the nozzle part 15, and the gap between the protrusions 31e and 31f and the inner wall surface of the nozzle body 10 (the inner wall surface 10a1 of the tip end part 10a) becomes narrower so that the rear support 2b does not pass through along the protrusions 31e and 31f and cannot escape from the gaps 31k and 31j within the tip end part 10a. As a result, the base end part 2b1 of the rear support 2b at the gap 31j abuts against the abutment surface 31s of the protrusion 31e and the inner wall surface 10a1 of the tip end part 10a, so that the frontward movement thereof in the optical-axis direction is restricted. Similarly, the distal end part 2b2 of the rear support 2b at the gap 31j abuts against the abutment surface 31s of the protrusion 31e and the inner wall surface 10a1 of the tip end part 10a, so that the frontward movement thereof in the optical-axis direction is restricted.

Subsequently, when the operator further pushes the plunger 30, the lens body 2a (and the front support 2b) is released out of the apparatus from the opening of the tip end part 10a of the nozzle body 10. At the time, the rear support 2b is still restricted by the protrusions 31e and 31f. When the operator further pushes the plunger 30, the restrictions on the rear support 2b are removed and the entire intraocular lens 2 is released into the eye from the opening of the tip end part 10a of the nozzle body 10.

This concludes the description of the behavior of the intraocular lens 2 when the intraocular lens insertion apparatus 1 is used.

Next, the effects of the intraocular lens insertion apparatus 1 according to the embodiment is described.

If the protrusions 31e and 31f are not provided, the base end part 2b1 and the distal end part 2b2 of the rear support 2b may exhibit abnormal behavior and be sandwiched between the outer peripheral surface of the working part 31 of the plunger 30 and the inner wall surface 10a1 of the tip end part 10a. In this case, the intraocular lens 2 may be damaged or the intraocular lens 2 may not be released into the eye.

Meanwhile, according to the embodiment, the position of the rear support 2b is stabilized by providing the protrusions 31e and 31f. Since the movement of the lens body 2a in the rotation direction N (see Fig. 1) around the base end part 2b1 of the rear support 2b as an axis is restricted, the behavior of the lens body 2a is also stabilized. The protrusions 31e and 31f are provided, so that the distal end part 2b2 of the rear support 2b may be prevented from being sandwiched between the outer peripheral surface of the working part 31 of the plunger 30 and the inner wall surface 10a1 of the tip end part 10a.

Since the plunger 30 has the protrusions 31e and 31f, when the plunger 30 moves into the tip end part 10a of the nozzle body 10, the protrusions 31e and 31f abut against the inner wall surface 10a1 of the tip end part 10a to stabilize the movement of the plunger 30.

Even when the lens body 2a (and the front support 2b) exits from the opening of the tip end part 10a of the nozzle body 10 as the plunger 30 is pushed in, the movement of the base end part 2b1, the intermediate part 2b3, and the distal part 2b2 of the rear support 2b is restricted (supported) by the inner wall surface 10a1 of the tip end part 10a, the protrusions 31e and 31f and the gaps 31j and 31k of the plunger 30. As a result, the intraocular lens 2 can be released into the eye with stable behavior.

Although the embodiment has been described as above, the structure of the intraocular lens insertion apparatus according to the embodiment is not limited by the content of the above description and various modifications may be made within the scope which does not lose consistency with the technical idea of the present invention. For example, the shapes and sizes of the protrusions 31e and 31f and the gaps 31j and 31k may be changed as appropriate according to the structure of the supports 2b for the intraocular lens 2, the nozzle part 15 and the tip end part 10a of the nozzle body 10.

Although in the foregoing description of the embodiment, a three-piece intraocular lens 2 is used, a one-piece intraocular lens having a lens body and supports which are integrally formed may be used instead. In this case, the positions of the gaps 31j and 31k provided in the working part 31 of the plunger 30 and the extending direction thereof to the notch 31c may be changed as appropriate. In the above description of the embodiment, it is assumed that the intraocular lens 2 is folded inward to have a trench shape at the tip end part 10a but may be folded outward have a ridge shape. This is applicable to the one-piece type intraocular lens. More specifically, the embodiment is applicable to the three-piece type intraocular lens as well as to the one-piece type intraocular lens and is also applicable to the case of inserting the lens into the eye in a ridge-shape folded state as well as in a trench-shape folded state. In the above description of the embodiment, the preset type intraocular lens insertion apparatus 1 is used, but the apparatus may be a so-called separator type intraocular lens insertion apparatus to which the operator sets the intraocular lens before surgery.

### [Reference Signs List]

- 1: Insertion apparatus
- 2: Intraocular lens
- 2a: Lens body
- 2b: Support
- 10: Nozzle body
- 10a: Tip end part
- 15: Nozzle part
- 30: Plunger
- 31: Working part
- 31c: Notch
- 31e, 31f: Protrusion
- 31j, 31k: Gap

## Claims

1. An intraocular lens insertion apparatus comprising:
a substantially tubular apparatus body which has, at a tip end thereof, an insertion tube part for inserting an intraocular lens in a folded state into an eye, the intraocular lens having a lens body and a long support which is connected at one end to the lens body;
a storage which stores the intraocular lens; and
an extruding member which slides in the apparatus body and moves the intraocular lens in the insertion tube part by pushing the intraocular lens, wherein
the extruding member has a tip end part having a first protrusion protruding to one side in a left-right direction and a second protrusion protruding to the other side in the left-right direction in a front view of the extruding member in a direction in which the intraocular lens is extruded,
during the movement of the intraocular lens in the insertion tube part by the extruding member,
the first protrusion restricts a base end part of the support from moving to a front side of the intraocular lens in an optical-axis direction by abutting against the base end part from the front side in the optical-axis direction, and
the second protrusion restricts a distal end part of the support nearer to a distal end thereof than the base end part from moving to the front side in the optical-axis direction by abutting against the distal end part from the front side in the optical-axis direction.

2. The intraocular lens insertion apparatus according to claim 1, wherein the extruding member has a notch positioned at a front side of the optical-axis direction than the intraocular lens stored in the storage, and
the notch supports an intermediate part of the support between the distal end part and the base end part from a rear side in the optical-axis direction in a state where the intraocular lens is stored in the storage.

3. The intraocular lens insertion apparatus according to claim 1 or 2, wherein in a state where the intraocular lens is stored in the storage,
the first protrusion is formed so that the base end part of the support can move to the front side in the optical-axis direction between the first protrusion and the apparatus body, and
the second protrusion is formed so that the distal end part of the support can move to the front side in the optical-axis direction between the second protrusion and the apparatus body.

4. The intraocular lens insertion apparatus according to any one of claims 1 to 3, wherein in a state where the tip end part of the extruding member is at the insertion tube part of the apparatus body,
a first gap through which the base end part of the support can be inserted is formed between the first protrusion and the insertion tube part, and
a second gap through which the distal end part of the support can be inserted is formed between the second protrusion and the insertion tube part, and
when the intraocular lens is moved into the insertion tube part from the storage by the extruding member, the base end part of the support enters the first gap, and the distal end part of the support enters the second gap.

5. The intraocular lens insertion apparatus according to any one of claims 1 to 4, wherein a width of a part where the first protrusion and the second protrusion are provided in the left-right direction is substantially equal to a width of at least a part of the insertion tube part in the left-right direction.

6. The intraocular lens insertion apparatus according to any one of claims 1 to 5, wherein the notch of the extruding member is provided nearer to a base end of the apparatus body than the first protrusion and the second protrusion.
